# EUROPÄISCHE PATENTSCHRIFT

(11) **EP 0 706 990 B1**
(45) Veröffentlichungstag und Bekanntmachung des Hinweises auf die Patenterteilung: **19.08.1998**
(21) Anmeldenummer: 95115503.5
(22) Anmeldetag: 30.09.1995
(51) Int. Cl.: C07C 67/58, C07C 69/54

(54) **Verfahren zur Abtrennung eines (Meth)acrylsäuremonoesters eines C4-bis C6-Alkandiols aus einer einen (Meth)acrylsäuremonoester eines C4- bis C6-Alkandiols und das C4- C6-Alkandiol enthaltenden wässrigen Lösung**
Process for the separation of a monoester of (meth)acrylic acid with a C4 to C6 alcanediol from an aqueous solution containing a monoester of (meth)acrylic acid with a C4 to C6 alcanediol and the C4 to C6 alcanediol
Procédé de séparation d'un monoester d'acide (méth)acrylique d'un alcanediol en C4 à C6 d'une solution aqueuse contenant un monoester d'acide (méth)acrylique d'un alcanediol en C4 à C6 et l'alcanediol en C4 à C6

(30) Priorität: 11.10.1994 DE 4436245
(43) Veröffentlichungstag der Anmeldung: 17.04.1996
(73) Patentinhaber: BASF AKTIENGESELLSCHAFT, 67056 Ludwigshafen (DE)
(72) Erfinder: Dockner, Toni, Dr., D-67149 Meckenheim (DE); Lermer, Helmut, Dr., D-67067 Ludwigshafen (DE); Rauh, Ulrich, D-67117 Limburgerhof (DE); Nestler, Gerhard, Dr., D-67061 Ludwigshafen (DE)

(56) Entgegenhaltungen:
- EP-A- 0 465 853
- DE-A- 1 518 572

## Beschreibung

Die vorliegende Erfindung betrifft ein Verfahren zum Abtrennen eines (Meth)acrylsäuremonoesters eines C₄- bis C₆-Alkandiols aus einer einen (Meth)acrylsäuremonoester eines C₄- bis C₆-Alkandicls und das C₄- bis C₆-Alkandiol enthaltenden wäßrigen Lösung durch Extraktion mit einem organischen Lösungsmittel.

"(Meth)acrylsäure" wird als verkürzte Schreibweise verwendet und steht für Acrylsäure oder Methacrylsäure.

(Meth)acrylsäuremonoester von C₄- bis C₆-Alkandiolen sind bekannt (vgl. z.B. Ullmann's Encyclopädie der technischen Chemie, Polyacryl-Verbindungen bis Quecksilber, Bd. 19, 4. Auflage, Verlag Chemie (1980), S. 10). Aufgrund ihrer Bifunktionalität (Hydroxylgruppe und monoethylenisch ungesättigte Gruppe) stellen sie interessante Ausgangsverbindungen dar. Beispielsweise eignen sie sich als vinylisch ungesättigte Comonomere in z.B. als Bindemittel geeigneten, durch radikalische Polymerisation erzeugten, Polymerisaten. Als alkoholische Verbindungen eignen sie sich aber auch z.B. für polymeranaloge Umsetzungen (z.B. Kondensationsoder Additionsreaktionen).

Es ist allgemein bekannt (z.B. DE-PS 1 518 572 und EP-A-465 853) (Meth)acrylsäuremonoester von C₄- bis C₆-Alkandiolen durch direkte Veresterung von (Meth)acrylsäure mit den entsprechenden Alkandiolen in Gegenwart saurer Veresterungskatalysatoren, unter Entfernung des entstehenden Wassers durch azeotrope Destillation mit einem geeigneten Schleppmittel, herzustellen. Da Alkohole unter dem Einfluß der sauren Veresterungskatalysatoren in der Regel zu Nebenreaktionen wie z.B. Etherbildung neigen, erfolgt die Veresterung vorzugsweise nur mit einem geringen Überschuß des Alkandiols. Unter solchen Bedingungen erfolgt normalerweise in nicht unerheblichen Mengen die Bildung der entsprechenden Diester, so daß als Produktgemisch in der Regel ein solches erhalten wird, das im wesentlichen aus
- Schleppmittel,
- Alkandiol,
- Monoester und
- Diester
besteht und aus dem der gewünschte Monoester abgetrennt werden muß.

Nachteilig für die geforderte Trennaufgabe ist, daß
- die Siedepunkte von Alkandiol, Monoester und Diester selbst unter reduziertem Druck bei erhöhten Temperaturen liegen;
- die Siedepunkte von Alkandiol, Monoester und Diester sehr nahe beieinander liegen;
- die vinylisch ungesättigten Monoester und Diester insbesondere in kondensierter Phase und bei erhöhter Temperatur eine erhöhte Polymerisationsneigung aufweisen.

Im Fall von Acrylsäure als Ausgangssäure und 1,4-Butandiol als Alkandiol betragen die relevanten Siedepunkte bei Normaldruck (1 atm) beispielsweise:
- 1,4-Butandiol: 230°C (Römpp Chemie Lexikon, 9. Auflage, Thieme Verlag, Stuttgart, 1989);
- 1,4-Butandiolmonoacrylat: ca. 230°C (Technische Information TI/ED 1331 d aus 1987, der BASF Aktiengesellschaft);
- 1,4-Butandioldiacrylat: ca. 225°C (Ullmanns Encyklopädie der technischen Chemie, Bd. 19, Verlag Chemie, Weinheim, 1980, S. 9).

Unter diesen Voraussetzungen scheidet eine rektifikative Auftrennung unter ökonomischen Gesichtspunkten faktisch aus (vgl. auch DE-OS 42 28 397, S. 1). In der DE-PS 15 18 572 wird zur Auftrennung des Produktgemisches empfohlen, selbiges mit Wasser zu versetzen und im Beisein der wäßrigen Phase den Diester mit einem Extraktionsmittel zu extrahieren. Vorzugsweise wird als Extraktionsmittel ein solches verwendet, das gleichzeitig als Schleppmittel für die Veresterung geeignet ist, um die den Diester enthaltende organische Extraktionsphase unmittelbar in die Veresterung rückführen zu können. Ansonsten wäre vor Anwendung des Extraktionsmittels das Schleppmittel vom Reaktionsgemisch gegebenenfalls destillativ abzutrennen. Extraktionsmittel, die gleichzeitig als Schleppmittel fungieren können, sind beispielsweise aliphatische und cycloaliphatische Kohlenwasserstoffe mit einem Siedepunkt von 65 bis 120°C (bei 1 atm) wie Hexan, Heptan, Cyclohexan oder Methylcyclohexan, wobei Cyclohexan besonders bevorzugt ist. Als wäßrige Phase fällt bei vorgenannter Extraktion eine wäßrige Lösung an, die im wesentlichen aus Wasser, Alkandiol und dem (Meth)acrylsäuremonoester des Alkandiols als gewünschtem Wertprodukt besteht.

Von dieser wäßrigen Lösung ausgehend, scheidet unter Berücksichtigung der beschriebenen Siedepunktslagen ihrer Bestandteile, eine rektifikative Trennung in Monoester und Alkandiol im wesentlichen aus. Es wäre zwar eine destillative Abtrennung des Wassers denkbar, dabei würde jedoch ein mit Alkandiol verunreinigter Monoester erhalten, der für viele Anwendungszwecke wenig brauchbar wäre (beispielsweise können Alkandiole bei radikalischen Polymerisationen aufgrund ihrer regelnden Wirkung das gewünschte Molekulargewicht beeinträchtigen oder bei Umsetzungen mit Diisocyanaten zu unerwünschter Vernetzung führen).

Die DE-PS 15 18 572 schlägt als Problemlösung vor, den Monoester aus der wäßrigen Lösung durch Extraktion mit einem geeigneten organischen Lösungsmittel abzutrennen und anschließend dieses Extraktionsmittel destillativ zu entfernen. Als solchermaßen geeignetes, die gestellte Aufgabe in befriedigender Weise lösendes Extraktionsmittel wird in der DE-PS 15 18 572 explizit ausschließlich Methylenchlorid aufgeführt. Nachteilig an einer Verwendung von Methylenchlorid ist jedoch seine nicht gegebene toxikologische Unbedenklichkeit.

In der EP-A 465 853 wird empfohlen, die Veresterung in Gegenwart von erheblichen Mengen an zugesetztem vorgebildetem Diester durchzuführen, um auf diese Weise den Anteil an nicht umgesetztem Alkandiol im Produktgemisch zu minimieren. Ferner empfiehlt die EP-A 465 853 aus dem Produktgemisch die katalytisch wirksame Säure auszuwaschen, danach in Abwesenheit von Wasser die Diesterverbindung mit entsprechenden Extraktionsmitteln, wie sie auch die DE-PS 15 18 572 empfiehlt, direkt zu extrahieren und den bei dieser Extraktionsweise in das Extraktionsmittel übergehenden Monoester im nachhinein aus selbigem mit Wasser auszuwaschen und die dabei anfallende wäßrige Phase mit der an Monoester reichenden Extraktionsphase zu vereinigen und den Diester in die Veresterung zurückzuführen. Hinsichtlich der wäßrigen, Monoester und geringe Mengen an Alkandiol enthaltenden Phase wird lediglich empfohlen, die leicht siedenden Bestandteile destillativ abzutrennen.

Nachteilig ist, daß auf diese Weise lediglich ein mit Alkandiol verunreinigter Monoester erhalten wird.

Die Aufgabe der vorliegenden Erfindung bestand daher darin, ein Verfahren zum Abtrennen eines (Meth)acrylsäuremonoesters eines C₄-bis C₆-Alkandiols aus einer einen (Meth)acrylsäuremonoester eines C₄- bis C₆-Alkandiols und das C₄- bis C₆-Alkandiol enthaltenden wäßrigen Lösung durch Extraktion mit einem von Methylenchlorid verschiedenen organischen Lösungsmittel zur Verfügung zu stellen, das in seiner extraktiven Wirkung dem Methylenchlorid im wesentlichen gleichkommt, jedoch ein reduziertes Gefährdungspotential aufweist.

Demgemäß wurde ein Verfahren zum Abtrennen eines (Meth)acrylsäuremonoesters eines C₄- bis C₆-Alkandiols aus einer einen (Meth)acrylsäuremonoester eines C₄- bis C₆-Alkandiols und das C₄-bis C₆-Alkandiol enthaltenden wäßrigen Lösung durch Extraktion mit einem organischen Lösungsmittel gefunden, das dadurch gekennzeichnet ist, daß als Extraktionsmittel ein organisches Lösungsmittel aus der Gruppe umfassend Ester aus C₁- bis C₄-Alkancarbonsäuren und C₁- bis C₅-Alkanolen, einfache und gemischte Dialkylether, die 4 bis 8 C-Atome aufweisen, sowie Gemische der genannten organischen Lösungsmittel, verwendet wird.

Bevorzugte erfindungsgemäße Extraktionsmittel sind Ester aus Essigsäure oder Propionsäure und C₃- bis C₅-Alkanolen, insbesondere C₄-Alkanolen, insbesondere Iso-Butylacetat. Ferner eignen sich vor allem Dialkylether die 4 bis 6 C-Atome aufweisen, insbesondere der Ether aus Methanol und tert.-Butanol. Des weitren sind Gemische der vorgenannten organischen Lösungsmittel als erfindungsgemäße Extraktionsmittel geeignet.

Die erfindungsgemäß zu verwendenden Extraktionsmittel sind dadurch ausgezeichnet, daß ihr Siedepunkt bei Normaldruck (in der Regel < 150°C) von demjenigen des extraktiv aufzunehmenden (Meth)acrylsäuremonoesters ausreichend verschieden ist, was eine nachfolgende einfache destillative Abtrennung des organischen Extraktionsmittels gewährleistet.

Ferner entspricht ihre extraktive Wirkung gegenüber den aufzunehmenden (Meth)acrylsäuremonoestern im wesentlichen derjenigen von Methylenchlorid. D.h. unter der Voraussetzung einer Anwendung identischer Volumina an Extraktionsmittel vermögen sie beim in Kontakt bringen mit einer wäßrigen, (Meth)acrylsäuremonoester eines C₄- bis C₆-Alkandiols enthaltenden Lösung aus selbiger bei 25°C im wesentlichen dieselbe Gleichgewichtsmenge an Monoester aufzunehmen wie Methylenclorid.

Eine weitere günstige Eigenschaft des erfindungsgemäßen Verfahrens ist, daß die erfindungsgemäß anzuwendenden Extraktionsmittel in mit Methylenchlorid vergleichbarem Ausmaß zwischen dem C₄- bis C₆-Alkandiol und dessen (Meth)acrylsäuremonoester zu selektieren vermögen.

Auch ist die Löslichkeit der erfindungsgemäß anzuwendenden Extraktionsmittel in Wasser in mit der entsprechenden Löslichkeit von Methylchlorid vergleichbarer Weise gering, was das Übergehen an Extraktionsmittel in die Wasser enthaltende Lösung minimiert.

Dieselben Relativverhältnisse gelten bei Betrachtung der Löslichkeit von Wasser in den erfindungsgemäß anzuwendenden Extraktionsmitteln bzw. Methylenchlorid.

M.a.W. ausgedrückt, vermögen die erfindungsgemäß zu verwendenden Extraktionsmittel das seitens des Standes der Technik für den selben Zweck empfohlene Methylenchlorid in überraschender Weise im wesentlichen wirkungsgleich zu ersetzen.

Bei Anwendung des Verfahrens der DE-PS 15 18 572 zur Herstellung von (Meth)acrylsäuremonoester eins C₄- bis C₆-Alkandiols fallen häufig wäßrige Lösungen an, die enthalten
- > 0 bis 15 Gew.-% C₄- bis C₆-Alkandiol,
- > 0 bis 15 Gew.-% (Meth)acrylsäuremonoester eines C₄- bis C₆-Alkandiols,
- bis zu 3 Gew.-% sonstige Verbindungen (z.B. (Meth)acrylsäure, katalytische Säure, Diester, Polymerisationsinhibitor, etc.) und
- als Restmenge Wasser.

Das erfindungsgemäße Verfahren eignet sich insbesondere zur extraktiven Abtrennung der (Meth)acrylsäuremonoester eines C₄- bis C₆-Alkandiols aus solchen vorgenannten Lösungen.

Dies gilt insbesondere dann, wenn die wäßrige Lösung im Rahmen einer entsprechenden Veresterung von 1,4-Butandiol und/oder 1,6-Hexandiol entstanden ist. Letztere Aussage trifft insbesondere dann zu, wenn es sich bei der zugehörigen Säure um Acrylsäure handelt.

Selbstverständlich eignet sich das erfindungsgemäße Verfahren aber auch zur extraktiven Abtrennung von (Meth)acrylsäuremonoester eines C₄- bis C₆-Alkandiols aus einer über den vorgenannten Monoester hinaus noch Wasser und C₄- bis C₆-Alkandiol enthaltenden Lösung, wie sie im Rahmen des Herstellverfahrens für (Meth)acrylsäuremonoester von C₄- bis C₆-Alkandiolen gemäß der EP-A 465 853 anfällt.

In der Regel wird man das erfindungsgemäße Verfahren bei einer Temperatur von 10 bis 60°C, vorzugsweise von 20 bis 40°C, besonders bevorzugt von 20 bis 30°C ausführen. Mit zunehmender Temperatur erhöht sich die Wahrscheinlichkeit für Nebenreaktionen. Die Wärmetönung im Verlauf der erfindungsgemäßen Extraktion ist von untergeordneter Bedeutung. Normalerweise wird die erfindungsgemäße Extraktion bei Normaldruck (1 atm) angewandt. Sie kann aber auch bei erhöhtem Druck (bis zu 15 atm Arbeitsdruck) angewendet werden.

Die erfindungsgemäße Extraktion kann in überraschender Weise sowohl im Fall einer sauren, einer neutralen oder einer basischen wäßrigen Ausgangslösung angewendet werden, ohne daß damit nennenswerte Verluste an erfindungsgemäßem Extraktionsmittel einhergingen, obgleich selbige z.B. gegenüber Hydrolyse oder ähnlichen Nebenreaktionen empfindlich sind.

In der Regel wird die wäßrige Ausgangslösung die im Rahmen der Veresterung eingesetzte katalytische Säure enthalten. Ein Teil derselben wird auch in die organische Extraktionsphase übergehen und in selbiger bei destillativer Abtrennung des Extraktionsmittels verbleiben. Stören solche Säureverunreinigungen im gewünschten (Meth)acrylsäuremonoester, können sie z.B. mit Wasser oder wäßriger Base ausgewaschen werden.

Zur Durchführung der erfindungsgemäßen Extraktion eignen sich die an sich üblichen Extraktionsvorrichtungen, insbesondere Füllkörperkolonnen. In selbigen werden Extraktionsmittel und wäßrige Lösung vorzugsweise im Gegenstrom geführt, wobei mit Vorteil die spezifisch schwerere Phase am Kopf der Kolonne aufgegeben wird. Selbstverständlich wird der erfindungsgemäße Verfahrensschritt ebenso wie alle übrigen Verfahrensschritte auf dem Weg zum (Meth)acrylsäuremonoester eines C₄- bis C₆-Alkandiols im Beisein üblicher Mengen üblicher Polymerisationsinhibitoren ausgeführt. Dies war auch in den nachfolgenden Beispielen so. Als solchermaßen zu verwendende Polymerisationsinhibitoren kommen insbesondere Phenothiazin, Hydrochinon sowie Hydrochinonmonomethylether in Betracht.

### Beispiele

(Alle Verfahrensschritte wurden im Beisein üblicher Mengen an Polymerisationsinhibitoren durchgeführt.)

100 ml einer sauren wäßrigen Lösung, die
- 6 Gew.-% 1,4-Butandiol,
- 8 Gew.-% 1,4-Butandiolmonoacrylat,
- < 3 Gew.-% sonstige Verbindungen und
- als Restmenge Wasser
enthielt, und im Rahmen der Herstellung von 1,4-Butandiolmonoacrylat gemäß der Verfahrensweise der DE-PS 15 18 572 angefallen ist, wurde bei 25°C und 1 atm mit jeweils 100 ml an verschiedenen Extraktionsmitteln in innigen Kontakt gebracht. Nach erfolgter Phasentrennung wurden im Extraktionsgleichgewicht bestimmt:
a) die Löslichkeit des Extraktionsmittels in der wäßrigen Phase (LE, Angabe in Gew.-% bezogen auf die wäßrige Phase);
b) die Löslichkeit von Wasser in der organischen Phase (LW, Angaben in Gew.-% bezogen auf die organische Phase);
c) die extraktive Wirkung gegenüber 1,4-Butandiolmonoacrylat, wobei der extraktiven Wirkung (der im Methylenchlorid gelösten Menge an 1,4-Butandiolmonoacrylat) von Methylenchlorid willkürlich der Wert 100 zugeordnet wurde (EW);
d) die Selektivität des Extraktionsmittels (S); zur Ermittlung von S wurde die im Extraktionsgleichgewicht befindliche organische Phase gaschromatographisch analysiert und in einfacher Weise das Gewichtsverhältnis der 1,4-Butandiolmonoacrylat (Zähler) sowie 1,4-Butandiol (Nenner) zuzuordnenden Flächenpeaks als Maß für S gebildet.

Die erhaltenen Ergebnisse, einschließlich der verwendeten Extraktionsmittel, weist die nachfolgende Tabelle aus. Sie enthält zusätzlich die Siedepunkte (Sdp., °C) bei 1 atm.

Im wesentlichen waren keine Nebenreaktionen der verwendeten Extraktionsmittel feststellbar.

**Tabelle**

| Extraktionsmittel | EW | S | LE | LW | Sdp |
|---|---|---|---|---|---|
| Methylenchlorid | 100 | 97/3 | 2 | 0,14 | 40,2 |
| Methyl-tert.-butylether | 95 | 92/8 | 4,8 | 1,1 | 55,2 |
| iso-Butylacetat | 100 | 97/3 | 0,7 | 1,6 | 118 |
| n-Butylacetat | 98 | 95/5 | 0,7 | 1,4 | 126,5 |
| Ethylacetat | 95 | 92/8 | 7,9 | 3,0 | 77,2 |
| Methylpropionat | 93 | 90/10 | 0,5 | - | 79,1 |
| Toluol (Vergleichsbsp.) | 51 | 98/2 | 0,005 | 0,05 | 110,6 |

## Patentansprüche

1. Verfahren zum Abtrennen eines (Meth)acrylsäuremonoesters eines C₄- bis C₆-Alkandiols aus einer einen (Meth)acrylsäuremonoester eines C₄- bis C₆-Alkandiols und das C₄- bis C₆-Alkandiol enthaltenden wäßrigen Lösung durch Extraktion mit einem organischen Lösungsmittel, dadurch gekennzeichnet, daß als Extraktionsmittel ein organisches Lösungsmittel aus der Gruppe umfassend Ester aus C₁- bis C₄-Alkancarbonsäuren und C₁- bis C₅-Alkanolen, einfache und gemischte Dialkylether, die 4 bis 8 C-Atome aufweisen, und Gemische aus solchen organischen Lösungsmitteln, verwendet wird.

2. Verfahren nach Anspruch 1, dadurch gekennzeichnet, daß als Extraktionsmittel ein organisches Lösungsmittel aus der Gruppe umfassend Ester aus Essigsäure oder Propionsäure und C₃- bis C₅-Alkanolen, einfache und gemischte Dialkylether, die 4 bis 6 C-Atome aufweisen, sowie Gemische der genannten organischen Lösungsmittel, verwendet wird.

3. Verfahren nach Anspruch 1 oder 2, dadurch gekennzeichnet, daß als Extraktionsmittel iso-Butylacetat, Methyl-tert.-butylether oder ein Gemisch der beiden vorgenannten organischen Lösungsmittel verwendet wird.

4. Verfahren nach Anspruch 1 bis 3, dadurch gekennzeichnet, daß 1,4-Butandiol oder 1,6-Hexandiol oder deren Gemisch das Alkandiol bilden.

5. Verfahren nach Anspruch 1 bis 4, dadurch gekennzeichnet, daß der abzutrennende (Meth)acrylsäuremonoester der Acrylsäuremonoester von 1,4-Butandiol, 1,6-Hexandiol oder von einem Gemisch dieser beiden Alkandiole ist.

6. Verfahren nach Anspruch 1 bis 5, dadurch gekennzeichnet, daß es bei einer Temperatur von 10 bis 60°C durchgeführt wird.

7. Verfahren nach Anspruch 1 bis 6, dadurch gekennzeichnet, daß es bei einer Temperatur von 20 bis 30°C durchgeführt wird.

8. Verfahren nach Anspruch 1 bis 7, dadurch gekennzeichnet, daß die wäßrige Lösung enthält:
- > 0 bis 15 Gew.-% C₄- bis C₆-Alkandiol,
- > 0 bis 15 Gew.-% (Meth)acrylsäuremonoester eines C₄- bis C₆-Alkandiols,
- bis zu 3 Gew.-% sonstige Verbindungen und
- als Restmenge Wasser.

9. Verfahren nach Anspruch 1 bis 8, dadurch gekennzeichnet, daß die wäßrige Lösung enthält:
- > 0 bis 15 Gew.-% 1,4 Butandiol,
- > 0 bis 15 Gew.-% 1,4-Butandiolmonoacrylat,
- bis zu 3 Gew.-% sonstige Verbindungen und
- als Restmenge Wasser.

10. Verfahren zur Herstellung eines (Meth)acrylsäuremonoesters eines C₄- bis C₆-Alkandiols durch Veresterung der (Meth)acrylsäure mit jenen Alkandiolen in Gegenwart saurer Veresterungskatalysatoren unter Entfernung des entstehenden Wassers durch azeotrope Destillation mit einem Schleppmittel, extraktive Auftrennung des Produktgemisches in eine den als Nebenprodukt entstehenden Diester enthaltende organische Lösung sowie eine verwendetes Alkandiol und (Meth)acrylsäuremonoester enthaltende wäßrige Lösung und anschließende extraktive Abtrennung des (Meth)acrylsäuremonoester aus der verwendetes Alkandiol und (Meth)acrylsäuremoncester enthaltenden wäßrigen Lösung mit einem Extraktionsmittel sowie nachfolgende Trennung von Extraktionsmittel und (Meth)acrylsäuremonoester, dadurch gekennzeichnet, daß als Extraktionsmittel für die Extraktion des (Meth)acrylsäuremonoesters aus der wäßrigen Phase ein organisches Lösungsmittel aus der Gruppe umfassend Ester aus C₁- bis C₄-Alkancarbonsäuren und C₁- bis C₅-Alkanolen, einfache und gemischte Dialkylether, die 4 bis 8 C-Atome aufweisen, und Gemische aus solchen organischen Lösungsmitteln, verwendet wird.

## Claims

1. A process for isolating a (meth)acrylic acid monoester of a C₄- to C₆-alkanediol from an aqueous solution containing a (meth)acrylic acid monoester of a C₄- to C₆-alkanediol and the C₄- to C₆-alkanediol by extraction with an organic solvent, wherein the extracting agent used is an organic solvent selected from the group consisting of esters of C₁- to C₄-alkanecarboxylic acids and C₁- to C₅-alkanols, symmetrical and unsymmetrical dialkyl ethers of 4 to 8 carbon atoms and mixtures of such organic solvents.

2. A process as claimed in claim 1, wherein the extracting agent used is an organic solvent selected from the group consisting of esters of acetic acid or propionic acid and C₃- to C₅-alkanols, symmetrical and unsymmetrical dialkyl ethers of 4 to 6 carbon atoms and mixtures of the stated organic solvents.

3. A process as claimed in claim 1 or 2, wherein the extracting agent used is isobutyl acetate, methyl tert-butyl ether or a mixture of the two abovementioned organic solvents.

4. A process as claimed in any of claims 1 to 3, wherein the alkanediol is 1,4-butanediol or 1,6-hexanediol or a mixture thereof.

5. A process as claimed in any of claims 1 to 4, wherein the (meth)acrylic acid monoester to be isolated is the acrylic acid monoester of 1,4-butanediol, of 1,6-hexanediol or of a mixture of these two alkanediols.

6. A process as claimed in any of claims 1 to 5, which is carried out at from 10 to 60°C.

7. A process as claimed in any of claims 1 to 6, which is carried out at from 20 to 30°C.

8. A process as claimed in any of claims 1 to 7, wherein the aqueous solution contains:
- > 0 to 15% by weight of C₄- to C₆-alkanediol,
- > 0 to 15% by weight of a (meth)acrylic acid monoester of a C₄- to C₆-alkanediol,
- up to 3% by weight of other compounds and
- water as the remaining amount.

9. A process as claimed in any of claims 1 to 8, wherein the aqueous solution contains:
- > 0 to 15% by weight of 1,4-butanediol,
- > 0 to 15% by weight of 1,4-butanediol monoacrylate,
- up to 3% by weight of other compounds and
- water as the remaining amount.

10. A process for the preparation of a (meth)acrylic acid monoester of a C₄- to C₆-alkanediol by esterification of the (meth)acrylic acid with those alkanediols in the presence of acidic esterification catalysts with removal of the resulting water by azeotropic distillation with an entraining agent, extractive separation of the product mixture into an organic solution containing the diester formed as a byproduct and an aqueous solution containing the alkanediol used and (meth)acrylic acid monoester and subsequent isolation of the (meth)acrylic acid monoester by extraction from the aqueous solution containing the alkanediol used and (meth)acrylic acid monoester with an extracting agent and subsequent separation of extracting agent and (meth)acrylic monoester, wherein the extracting agent used for the extraction of the (meth)acrylic monoester from the aqueous phase is an organic solvent selected from the group consisting of esters of C₁- to C₄-alkanecarboxylic acids and C₁- to C₅-alkanols, symmetrical and unsymmetrical dialkyl ethers of 4 to 8 carbon atoms and mixtures of such organic solvents.

## Revendications

1. Procédé pour la séparation d'un mono(méth)-acrylate d'un alcanediol en C₄-C₆ à partir d'une solution aqueuse contenant un mono(méth)acrylate d'un alcanediol en C₄-C₆ et l'alcanediol en C₄-C₆, par extraction avec un solvant organique, caractérisé en ce que l'on utilise comme agent d'extraction un solvant organique choisi dans l'ensemble comprenant des esters d'acides alcane(C₁-C₄)carboxyliques et d'alcanols en C₁-C₅, des éthers dialkyliques simples et des éthers dialkyliques mixtes, qui comportent de 4 à 8 atomes de carbone, et des mélanges à base de ces solvants organiques.

2. Procédé selon la revendication 1, caractérisé en ce que l'on utilise comme agent d'extraction un solvant organique choisi dans l'ensemble comprenant des esters d'acide acétique ou d'acide propionique et d'alcanols en C₃-C₆, des éthers dialkyliques simples et des éthers dialkyliques mixtes, qui comportent de 4 à 6 atomes de carbone, ainsi que des mélanges des solvants organiques cités.

3. Procédé selon la revendication 1 ou 2, caractérisé en ce que l'on utilise comme agent d'extraction l'acétate d'isobutyle, l'éther méthyl-tert-butylique ou un mélange des deux solvants organiques précités.

4. Procédé selon l'une des revendications 1 à 3, caractérisé en ce que le 1,4-butanediol ou le 1,6-hexanediol ou un mélange de ceux-ci constitue l'alcanediol.

5. Procédé selon l'une des revendications 1 à 4, caractérisé en ce que le monoester d'acide (méth)acrylique à séparer est le monoacrylate de 1,4-butanediol, de 1,6-hexanediol ou d'un mélange de ces deux alcanediols.

6. Procédé selon l'une des revendications 1 à 5, caractérisé en ce qu'il est effectué à une température de 10 à 60°C.

7. Procédé selon l'une des revendications 1 à 6, caractérisé en ce qu'il est effectué à une température de 20 à 30°C.

8. Procédé selon l'une des revendications 1 à 7, caractérisé en ce que la solution aqueuse contient:
- > 0 à 15 % en poids d'alcanediol en C₄-C₆,
- > 0 à 15 % en poids de mono(méth)acrylate d'un alcanediol en C₄-C₆,
- jusqu'à 3 % en poids d'autres composés et
- de l'eau comme quantité restante.

9. Procédé selon l'une des revendications 1 à 8, caractérisé en ce que la solution aqueuse contient:
- > 0 à 15 % en poids de 1,4-butanediol,
- > 0 à 15 % en poids de monoacrylate de 1,4-butanediol,
- jusqu'à 3 % en poids d'autres composés et
- de l'eau comme quantité restante.

10. Procédé pour la préparation d'un mono(méth)acrylate d'un alcanediol en C₄-C₆, par estérification de l'acide (méth)acrylique par les alcanediols en présence de catalyseurs acides d'estérification, avec élimination de l'eau formée, par distillation azéotrope avec un agent d'entraînement, séparation par extraction du mélange constituant le produit, en une solution organique contenant le diester formé en tant que sous-produit ainsi qu'une solution aqueuse contenant le monoester d'acide (méth)acrylique et l'alcanediol utilisé, et séparation subséquente du monoester d'acide (méth)acrylique par extraction à partir de la solution aqueuse contenant le monoester d'acide (méth)acrylique et l'alcanediol utilisé, à l'aide d'un agent d'extraction, ainsi que séparation subséquente de l'agent d'extraction et du monoester d'acide (méth)acrylique, caractérisé en ce que, pour l'extraction du monoester d'acide (méth)acrylique à partir de la phase aqueuse, on utilise comme agent d'extraction un solvant organique choisi dans le groupe comprenant des esters d'acides alcane(C₁-C₄)carboxyliques et d'alcanols en C₁-C₅, des éthers dialkyliques simples et des éthers dialkyliques mixtes, qui comportent de 4 à 8 atomes de carbone, et des mélanges à base de tels solvants organiques.
